# EUROPEAN PATENT APPLICATION

(11) **EP 4 413 929 A1**
(43) Date of publication of application: **14.08.2024**
(21) Application number: 23305177.0
(22) Date of filing: 09.02.2023
(51) Int. Cl.: A61B 6/03, A61B 6/00, G06F 3/04847, G16H 40/63

(54) **VASCULATURE-BASED INTERACTIVE BOLUS ROI PLACEMENT AND SCAN PLANNING USING 3D CT SURVIEWS**

(71) Applicant: Koninklijke Philips N.V., 5656 AG Eindhoven (NL)
(72) Inventor: DESHPANDE, Hrishikesh Narayanrao, 5656 Eindhoven (NL); SCHMITT, Holger, 5656 Eindhoven (NL); VLACHOMITROU, Anna Sesilia, 5656 Eindhoven (NL); NEMPONT, Olivier, 5656 Eindhoven (NL)
(74) Representative: Philips Intellectual Property & Standards

(57) **Abstract**

A system (FS) and related method for facilitating contrast-agent based tomographic imaging.. A graphics display generator (GDG) generates a graphics display (GD) of a graphical user interface (GUI) for display on a display device (DD). The graphics display (GD) may include a visualization of a segmentation in a 3D surview image, and of a slider element (SL) indicative of a reference location (m-ROI) along the segmentation. The reference location may be used for monitoring in a contrast agent assisted imaging phase for presence of contrast agent in relation to a target anatomical feature (TAF). An event handler (EH) may instruct graphics display generator (GDG), upon receiving user input, to update the graphics display (GD), so as to cause the slider element (SL) to slide along the segmentation, the slider thereby indicating different such one or more reference locations.

## Description

### FIELD OF THE INVENTION

The invention relates to a system for facilitating contrast-agent based tomographic imaging, to a related method, to an imaging arrangement, to a computer program element, and to a computer readable medium.

### BACKGROUND OF THE INVENTION

2D (two dimensional) projection surview image(s) acquired before diagnostic CT (computed tomography) imaging plays an important role in assisting clinical technologists ("user"), for example in proper patient positioning or in achieving optimal image quality.

In addition to conventional 2D surviews (such as frontal and sagittal), today it is possible to acquire 3D (reconstructed) surview images (an image volume) at clinically acceptable dose. They offer several advantages over conventional 2D surviews, and open up new possibilities for CT workflow optimization, which was not possible earlier.

It is common practice in CT imaging to perform a 2D surview scan covering the entire area of interest including an expected "bolus" tracking location. Bolus is a volume of administered contrast agent material such as Iodine. Contrast agent may be used in order to boost image contrast of weakly radiation absorbing anatomical structures of interest, such as cardiac vessels in cardiac imaging. The user then manually selects the z-position on such 2D surview(s), and the corresponding axial image, also known as a "locator", is acquired and reconstructed for definition ("placement") of a region of interest ("ROI") for bolus tracking or monitoring for bolus arrival. Bolus travels with blood flow and accumulates, for a time, at the anatomy of interest. It is an object to trigger imaging at the right moment to capture imagery at high contrast before bolus wash-out.

Multiple locator scans may be needed to find proper location for the tracking ROI. The ROI to be captured for tracking the bolus is placed within this locator slice, and is monitored for the arrival of the contrast agent bolus, by repeated acquisition of this single axial slice. The subsequent diagnostic CTA (CT angiography) acquisition is triggered when a threshold value of contrast induced density is reached within this ROI.

A major disadvantage of bolus tracking using conventional 2D surview images, or of bolus tracking in general, is that planning of bolus tracking during the scan procedure is complex and relies heavily on clinical user's skill and experience. The process is error prone. For example, there are several manual interventions needed by user, such as manually selecting the z-slice, placing an ROI on the acquired locator slice etc. If there are human errors in the localization of anatomical structures and placing the ROI in the localizer image, the diagnostic CT scan may be started at a suboptimal time. This could subsequently result in poor image quality and wrong diagnosis, and could lead to unnecessary readministration of contrast agent and image retakes, with potential side-effects on patient and/or medical staff. Also, the manual localization process is subject to intra- and inter-individual variability. Another disadvantage is the need to acquire special locator scans(s) which requires extra time and dose.

### SUMMARY OF THE INVENTION

There may therefore be a need for improvements in efficient operation of an imaging apparatus. In particular, there may be a need for establishing reliably the correct timing for triggering an imaging operation for such an imaging apparatus.

An object of the present invention is achieved by the subject matter of the independent claims where further embodiments are incorporated in the dependent claims. It should be noted that the following described aspect of the invention equally applies to the related method, to the image arrangement, to the computer program element and to the computer readable medium.

According to a first aspect of the invention there is provided a system for facilitating contrast-agent based tomographic imaging, the system comprising, when in use:
an input interface for receiving a 3D surview image volume acquired by a tomographic imaging apparatus of at least a part of a patient in a preparatory phase prior to a contrast agent assisted imaging phase, the 3D surview image volume including a segmentation (s) for a vessel through which contrast agent is to pass in the later contrast agent assisted imaging phase;
a graphics display generator configured to generate a graphics display of a graphical user interface for display on a display device, the graphics display including a visualization of the said segmentation and of a slider element indicative of a reference location along the segmentation, the reference location suitable for monitoring in the later contrast agent assisted imaging phase for presence of contrast agent in relation to at least one target anatomical feature; and
an event handler configured to instruct the graphics display generator, upon receiving user input, to update the graphics display, so as to cause the slider element to slide along the segmentation, the slider thereby indicating different such one or more reference locations.

The segmentation is preferably computed/derived from the 3D surview image. Machine learning (deep learning model) may be used for example.

In embodiments of the system, the graphics display generator is operable to adapt a spatial extent (eg, shape, orientation, size) of the slider element to correspond to, and vary with, a geometrical aspect (eg, cross-section) of the segmentation at the said different reference locations.

In embodiments the graphics display generator is operable to lock the slider element onto a direction defined by the segmentation. This allows assisting user to define a clinically meaningful monitoring location. Operation is less "fiddly" as correct bolus monitoring definition may be otherwise difficult, in particular in stress situations.

In embodiments, the said direction is as per a center line of the segmentation.
In embodiments, the visualization of the segmentation is configured to distinctly represent a wall portion of the vessel. For example, the wall portions may be highlighted by color or grey value modulation, may be rendered in dash-lines, heavy lines, in different color against background and/or interior of segmentation, etc. The distinct visualizations of the wall portions may be modulated to distinguish between calcified or non-calcified portions, or between various levels of calcifications. This helps user avoiding placing monitoring ROI at calcified or heavily calcified portions of the vessel as such portions have been found to have lesser utility for bolus monitoring purposes. For better calcification definition, spectral imaging may be used when recording the surview image.

In embodiments, the graphics display generator is operable to cause the graphics display to include information on i) a distance of the at least one target anatomical feature to a) the reference location of the vessel that corresponds to the current slider location, and/or b) to one or more anatomical landmarks, and/or ii) estimated contrast agent arrival time at the said reference location.

In embodiments the system may include an output interface for passing on a user selected one of the one or more reference locations to the imaging apparatus for demarking one end of a subset in image domain (as referred to as "scan box") in respect of which projection data is to be collected by the imaging apparatus in the imaging phase.

In embodiments the graphics display generator is operable to generate the graphics display to further include a visualization of the said subset, with the slider element positioned at the said one end of the visualization of the subset.

In embodiments the system may include an output interface for passing on a user selected one of the one or more reference locations to instruct the imaging apparatus to acquire a first set of projection data whilst contrast agent propagates in patient, and comprising a reconstructor to reconstruct, based on the projection data and at a first image quality, sectional tracker imagery in a plane passing through the selected reference location.

In embodiments the arrival time is based on hemodynamical modelling.

In embodiments, the said vessel includes at least a part of the aorta.

The facilitator system thus affords a user interface functionality. The user of the facilitator system may be used in conjunction, and cooperation with, a fully automatic segmentor system [machine leaning ("ML) based or other]. Whilst such segmentor systems may be able in some cases to find completely satisfactory suggestions for an adequate bolus monitoring region definition, in many cases it is still beneficial if human clinical user can still fine-tune the solution proposed by such automated segmentation system. Thus, the proposed user interface functionality provides an intuitive user input solution that allows user to perform this fine-tuning task quickly and safely with good results to ultimately locate an appropriate bolus monitoring region. In particular, and as an example, the proposed interface functionality allows visualizing results from, in particular (but not only), ML-based solutions, and perform such fine-tuning modifications when deemed necessary by user.

The upshot of the above is that the proposed interface functionality facilitates achieving a more accurate and reliable way to establish the correct timing for diagnostic projection data acquisition. The likelihood for image retakes can be reduced, which yields lower dose exposure to staff and patient, higher imaging throughput, and lower machine wear and tear, the latter being a specific concern in X-ray imaging where certain machine parts, such as the anode disk, are exposed to significant temperature gradients during data acquisition.

The proposed user functionality strikes a useful balance between affording to the user liberty in fine-tuning an initial location for the monitoring region, combined with a set of useful, clinically motivated, set of restrictions that provide meaningful guidance function. The user, even the novice or under-stress clinical user, arrives at an appropriate result for m-ROI definition quicker and safer.

Whilst the proposed user functionality can be used in conjunction with automatic segmentors as mentioned above, such automated systems are not a necessity herein. Thus, the proposed user functionality can be used without such automated segmentor systems, and can be used instead to review manually segmented m-ROIs, of the same user or of a different user for example, as required. Use with fully automatic, semi-automatic or manual segmentors are envisaged, although the proposed user interface functionality may be of particular benefit with fully automatic or semi-automatic segmentors.

The proposed system allows for a higher degree of interaction in the planning of bolus tracking ROIs, as compared to fully automated systems. Thus, what is proposed herein in embodiments is a vasculature-based interactive bolus ROI placement and scan planning method based on 3D CT Surviews. The proposed user functionality is geared towards placement of bolus monitoring ROIs in 3D surview scans. Working in 3D surview imagery, as opposed to mere 2D surview data affords overcoming the following disadvantages to be found in dealing with 2D surview image data:-
2D surviews are associated with an inherent disadvantage that the accurate 3D distance between the bolus ROI and the target anatomy cannot be computed, thus not allowing the estimation of the time required for the contrast agent to reach the target anatomical region. This often leads to larger amount of contrast administration and the images with sub-optimal image quality;

Placement of bolus ROI during scan planning is a complex task and relies heavily on clinician expertise;

Special locator scans (single z-slices) need to be acquired which requires extra time and dose to the patient. These slices are automatically obtained as 3D surviews already cover the field of view with axial slices;

Human user errors in the localization of anatomical structures and placing the ROI in the localizer image could lead to starting the CT scan at a suboptimal time. This could subsequently result in poor image quality and could lead to a wrong diagnosis, image re-acquisitions, patient recalls and unnecessary administration of contrast agent with potential side-effects.

With the proposed system, a user- interactive way of placing m-ROI is provided, which enhances user convenience and allows user to place m-ROI as per his or her preferences.

In the proposed system, a segmentation of the vessel through which the bolus is expected to travel is provided. The vessel may be the whole or part of the aorta for example, or may be a partial or full segmentation of any other vessel system of interest. The segmentation of the vessel pathway for the bolus helps user in visualizing the relative location of the aortic walls in comparison with the placed or moved bolus ROI symbol (such as circle, ellipse, etc). The vessel (eg aortic) walls are explicitly presented to the user in addition to the placed bolus m-ROI in preferred embodiments. Such system also allows for example the user to visualize calcifications in the aorta and place the bolus m-ROI accordingly, in particular by avoiding areas of calcifications in the selected m-ROI. Material selective imaging, such as spectral imaging, may be of particular benefit in this regard.

The proposed interactive bolus m-ROI placement system and the suggested visualization techniques assist user in better placing the bolus m-ROI in relation to the other important anatomical landmarks. For example, the landmarks may include one or more various sub-sections of the vessel, such as of the aorta. The subsections may include aortic arch ascending and descending aorta, etc. The vessel section landmarks could be labelled or highlighted differently, thus giving user more control when placing the bolus m-ROI in relation to prominent anatomical landmarks and enabling user in making a more informed decision. In addition or instead, the visualization can also assist the user in providing information on distance and transit time estimations relative to one or more landmarks.

The proposed system and related method (see below) allow user to better adjust/modify a given m-ROI placement definition along the center-line of the vessel, such as of the aorta, while using the distances to anatomical landmarks and visualization of the aortic walls/radius.

In yet another aspect there is provided an imaging arrangement comprising the system of any one of the above mentioned embodiments, and one of more of: the imaging apparatus, the display device, a contrast agent administration apparatus for administering contrast agent, a segmentor configured to provide the segmentation of the surview image.

In further embodiments, the imaging arrangement may further include a bolus monitoring system. The bolus monitoring system may be configured for in-image monitoring to perform monitoring based on the tracker imagery in a pre-defined image-neighborhood in the tracker imagery around the reference location. In embodiments, the bolus monitoring system may be configured, via a controller, to instruct imaging apparatus to acquire, at a second image quality higher than the first image quality, a second set of projection data upon the in-image monitoring unit issuing a trigger signal based on the monitored one or more image values in the image-neighborhood.

In another aspect there is provided a method for facilitating contrast-agent based tomographic imaging, comprising:
receiving a 3D surview image volume acquired by a tomographic imaging apparatus of at least a part of a patient in a preparatory phase prior to a contrast agent assisted imaging phase, the 3D surview image volume including a segmentation (s) for a vessel through which contrast agent is to pass in the later contrast agent assisted imaging phase;
generating a graphics display of a graphical user interface for display on a display device, the graphics display including a visualization of the said segmentation and of a slider element indicative of a reference location along the segmentation, the reference location suitable for monitoring in the later contrast agent assisted imaging phase for presence of contrast agent in relation to at least one target anatomical feature; and
instructing the graphics display generator, upon receiving user input, to update the graphics display, so as to cause the slider element to slide along the segmentation, the slider thereby indicating different such one or more reference locations.

In yet another aspect there is provided a computer program element, which, when being executed by at least one processing unit, is adapted to cause the processing unit to perform the method.

In yet another aspect there is provided at least one computer readable medium having stored thereon the program element.

The system and related methods (see below) are mainly envisaged herein for use in the medical realm. However, the principles described herein may also be used in fields of endeavor outside the medical field, such as in contrast agent (eg, dye) assisted examination of inaccessible hydraulic or plumbing systems, or in hydrological exams to understand subterranean water movements, etc. As is the case for medical imaging, the system and method allow reducing wear and tear effects on imaging equipment as they can be used with better timing to trigger acquisitions at the instant of best image harvest, thus avoiding repeated imaging.

*"user"* relates to a person, such as medical personnel or other, operating the imaging apparatus or overseeing the imaging procedure. In other words, the user is in general not the patient.

*"object"* is used herein in the general sense to include animate "objects" such as a human or animal patient, or anatomic parts thereof but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient.

*"surview image"* is used herein is a 3D image volume obtained at lower radiation dose cost and at a lager field-of-view (FOV) than dose cost and FOV for and of a subsequent diagnostic image volume. The diagnostic image volume is required to accomplish or support a medical task, such as diagnosis, therapy, or other. The surview image is not in general used for such tasks, but may be used in planning for example. The FOV of the surview image volume includes not only the anatomy of interest, which is the very object of the medical task, but in general also captures other landmark anatomies that have only an auxiliary function for the imaging, but in general not for the medical task. The FOV may be a whole-body scan, but this needn't be so in all cases. In general, the FOV of the surview image captures larger anatomic section, such as abdominal, thorax, etc.

*"z-position"* or similar terminology as used herein relates to one example of a reference location in image domain as envisaged herein for definition of the tracker imagery. "z-position" in general relates to position on one (the "Z-axis") of the 3 spatial coordinate axes that span image domain. In general, this Z-axis corresponds to the rotation axis of rotational tomographic imagers, or to the imaginary rotation axis in imagers of 5^{th} generation scanners. However, the said Z-axis may differ from the rotation axis for reformatted surview volumes, also envisaged herein.

*"image processing",* in this connection, it is observed that any processing of imagery, envisaged and described herein, such of the surview image or segmentation of vessel, landmark, m-ROI etc, in whichever embodiment and configuration or setup, includes, not only processing in image domain of image values (eg, HU values etc), but such reference specifically include processing in transform domain, such as frequency domain, or any other domain in which the surview image is transformed first, and at least a part of the processing takes place there, with optional re-transforming into image domain, as required. Transforms envisaged herein include any Fourier based transform (Laplace transform, Discrete cosine transform, etc), Wavelet- transform, Hilbert transforms, Haar transforms, Distance Transform, etc.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of a medical imaging arrangement;
Fig. 2 illustrates a contrast agent-based imaging protocol;
Fig. 3 shows steps of a facilitator system for facilitating contrast agent assisted tomographic imaging;
Fig. 4 shows a block diagram of a facilitator system using a graphical display generator as envisaged herein in embodiments;
Fig. 5 shows a schematic block diagram of the graphics display generator of the facilitator system of Fig. 4;
Fig. 6 shows an illustration of a graphics display generatable by the systems of Figs 4, 5;
Fig. 7 shows another illustration of the graphics display according to one optional embodiment; and
Fig. 8 shows a flow chart of a computer implemented method for graphical user interface-assisted contrast agent based imaging.

### DETAILED DESCRIPTION OF EMBODIMENTS

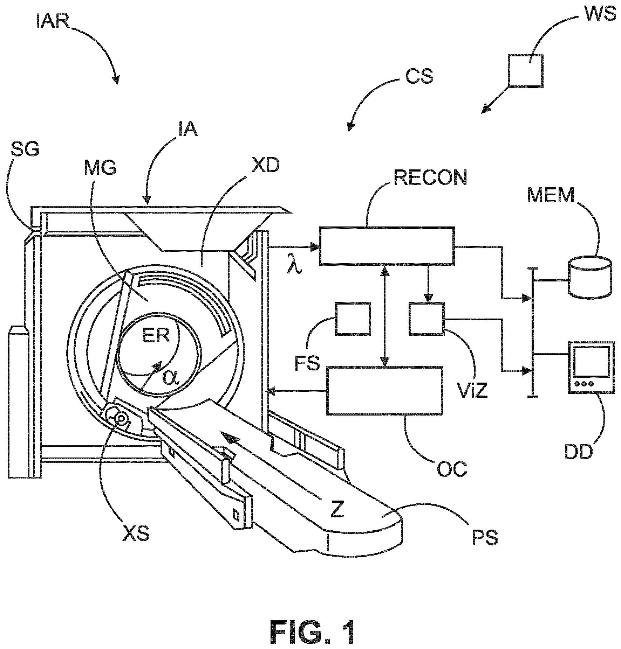

Reference is first made to Fig. 1 which shows a schematic block diagram of a medical imaging arrangement MAR as envisaged herein in embodiments. The arrangement IAR may include a medical imaging apparatus IA ("imager" for short), preferably of the tomographic X-ray based type, Thus the imager may be computed tomography (CT) scanner, but interventional systems such as those of the C-arm/U-arm-type are not excluded herein. Other tomographic modalities such as MRI, PET and others still are not excluded herein in some other embodiments. The medical imaging arrangement is preferably envisaged for contrast agent-based imaging protocols, such as angiography.

The arrangement IAR further includes a computing system CS which is broadly operable to process data, including image data, as provide by the imager. The computing system may further allow controlling operation of the imager. The computing system may be remotely located from the imaging apparatus IA, or may located close thereto, such as integrated into an operator console CS from which a user can operate the imaging apparatus, in particular can control an imaging operation to obtain medical imagery for diagnosis, treatment, planning (such as in radiation therapy, or other), etc.

Broadly, and as will be explained more fully below, the computing system CS includes a facilitator system FS that may be used to establish the right timing to trigger imaging operation to ensure good quality (contrasted) imagery is obtained. In addition, or instead, the facilitator system FS supports a user input UI functionality that allows clinical user to adjust, fine-tune, or to define from scratch, a contrast agent bolus monitoring region ("m-ROI") at which contrast agent accumulation is to be monitored for to accurately establish the said timing for triggering image operation in respect to a target anatomical feature TAF. The computing system CS., in particular its facilitator system FS, may be implemented as a Cloud solution, run on one or more servers. The imaging apparatus IA may be installed in a clinical facility, such as in a hospital for example. The computing system may be installed or used in a control room next to the imaging room where the imager IA is located. In some embodiments the computing system may be integrated into the imager IA. The imager IA may be communicatively coupled via a wired or wireless (or partly both) communication channel CC to computing system CS. The computing system CS may be arranged as a stationary computing system, such as a desktop computer, or as said server, or may be arranged as a mobile device, such as a laptop, smartphone, tablet, etc. For example, the computing system CS may be arranged on a work station WS associable with the imager IA.

Before explain operation of the facilitator system FS in more detail, reference is first mode to components of the imager IA which will the following explanations in relation to the facilitator system FS.

The imaging apparatus IA is operable to generate, in particular acquire, projection data λ which are forwarded through communication channel to the computing system CS for reconstruction into tomographic (sectional) images. The computing system CS runs one or more reconstructor units RECON, which implement one or plural reconstruction algorithms. In general, reconstruction algorithms implement a mapping that maps projection data λ located in projection domain into image domain. Image domain is a portion of 3D space and is located in the examination region ER of the imaging apparatus, whilst the projection domain is in 2D, and is located at an (X-ray) detector XD of the imaging apparatus IA.

As said, the imaging apparatus IA is preferably of the tomographic type, and is preferably configured for multi-directional projection image acquisition. The imaging apparatus IA is thus capable of acquiring projection images λ along different projection directions α relative to the examination region ER, and thus the anatomical region ("ROI") of interest of the patient. Acquisition be done in embodiments by a rotational system where at least the X-ray source XS is arranged in a moveable gantry MG.

The movable gantry (and with it the X-ray source XS in embodiments) is rotatable in a stationary gantry SG around the examination region ER in which the patient/ROI resides during imaging. Opposite the X-ray source in the movable gantry there is the X-ray detector XD which may rotate with the gantry and X-ray source around the examination region ER to realize the different projection directions α.

As schematically indicated in Fig. 1, patient's longitudinal or imaging axis Z may extend into the examination region ER during imaging. Patient PAT may lie on a patient support PS, such as a bed, which is positioned at least partly in the examination region ER during imaging. In some, but not all embodiments, helical imaging protocols are envisaged herein where there is a relative lateral motion along the longitudinal axis Z between X-ray source XS and the patient PAT. For example, the patient support PS may be advanced through the examination region ER during the multi-directional projection image acquisition, for example during rotation of the X-ray source XS around the patient.

The CT scanner setup as illustrated in the Fig. 1 is merely according to one embodiment, and other tomographic imaging equipment, such as C-arm or U-arm scanners, Cone beam CT arrangements, mammographic imagers, etc, are not excluded herein. C-arm cone beam imagers are preferred herein in some embodiments. In addition, the multi-directional acquisition capability may not necessarily result from a rotational system such as shown in Fig. 1. Non-rotational imaging systems are also envisaged, such as in CT scanners of 4^{th} or 5^{th} generation, where multiple X-ray sources are arranged around the examination region in a source annulus for example. In addition or instead, the detector XD may be arranged as a detector annulus around the examination region. Thus, in such systems there is no rotation of the X-ray source XS or detector XD, or of both.

There may be an operator console OC through which a user, such as medical personnel, controls imaging operation. For example, user may request initiating the image acquisition or may request reconstruction or other operations, or may initiate transmission of data to the computing system CS, or may halt such transmission as required.

During imaging, an X-ray beam XB issues forth, along the different projection directions α, from a focal spot of the X-ray source(s) XS. The beam XB passes through the examination region with the patient in it. The x-radiation interacts with patient tissue. The X-ray beam XB is modified as a result of this interaction. In general, such X-ray beam XB modification includes attenuation and scattering of the original incoming X-ray beam. The modified X-radiation is then detected as a spatial distribution of various intensities at X-ray sensitive pixels of the detector XD.

It is not necessary herein to acquire projection images λ over a full 360° angular range around the examination region ER. Acquisition over a partial angular range such as a range of 270°, 180°, or even less may be sufficient. The X-ray detector is preferably configured for acquisition of 2D projection images with rows and columns of intensity values as registered by the detector pixels. That is, the detector pixels themselves may be arranged in a matrix layout. Such a 2D layout may be used in divergent imaging geometries, such as cone or fan beam or others. However, one dimensional detector pixel layouts (such as along a single line) are not excluded herein, and neither are parallel beam geometries.

The reconstructor RECON implements one or more reconstruction algorithms to process the projection imagery. Specifically, the reconstructor RECON may compute sectional image V of the examination region (with the patient in it) for diagnostic, therapeutic or other purposes. The reconstructor RECON may be able to produce sectional volumetric image data ("image volume(s)") V. However, this does not exclude producing a single image slice in the examination region as required. Thus, the reconstructed imagery may be denoted herein as V which may include the whole volume, a partial volume or a specific section therethrough. Volumetric reconstruction may be facilitated by the helical movement and/or the 2D layout of the X-ray detector XD. A scan box SB may be defined by user, in particular by using the user input UI functionality as supported by facilitator system FS. The scan box CB is a portion of space in image domain. It is thus a 3D object, but may be visualized in 2D view as a rectangular, hence the name scan box. The scan box defines the volume which is to be scanned in order to acquire projection data from which the volume of space demarked by the scan box is to be reconstructed. The scan box in general includes the monitoring region m-ROI, the target anatomical feature TAF, and, optionally, one or more anatomical landmarks LM.

The reconstructed volumetric imagery *V* may be stored in a memory MEM or may be otherwise processed, as required. The reconstructed volumetric imagery *V* may be visualized by a visualizer VIZ. The visualizer VIZ may generate a graphics display of the volume or a given slice. The graphics display may be displayed on a display device DD. The visualizer VIZ may map a section of image volume V, or the image volume V as a whole, onto a grey value or a color palette. The visualizer VIZ may control, via suitable interfaces, video circuitry to effect display of the graphics display on the display device DD. In addition or instead of so displaying, the reconstructed imagery *V* may be stored in a memory for later review or for other types of processing. Such a memory may include an image repository, such as a database (eg, PACS), or other (preferably) non-volatile data storage arrangement.

The reconstructed volume imagery *V* may be manipulated, for example by reformatting, to define cross-sectional planes that are different from the sectional plane defined by the imaging geometry. Such reformatting may allow medical user to better discern tissue type or anatomic details of the internals of the patient, in dependence on the medical purpose at hand, such as for diagnosis or preparation for some type of treatment, etc.

The so reconstructed volume *V* may be referred to herein as the target image acquired in a target or operational phase of the contrast agent assisted imaging procedure. This reconstructed image V is envisaged as a diagnostic image that represents the target anatomical feature TAF, such as a target anatomy, an organ, part of an organ, or group of organs, different tissue types, etc, and at sufficient contrast to safely inform therapeutic or diagnostic decisions, or other medical decisions such as to have further imaging sessions done, possibly using other imaging modalities, or other tasks (tests, etc) that need be done in light of the imagery.

The (target) projection imagery λ, from which the target volume V is reconstructed, is acquired at a sufficient dosage by suitably controlling via operator console OC the dose to be used. This may be done by controlling the voltage and/or amperage setting of the tube of the x-ray source XS to ensure a certain diagnostic image quality. As mentioned earlier, the imaging protocol envisaged herein is preferably contrast agent based to ensure that the target anatomical feature TAF, that may be natively of low radio- opacity, can still be imaged at sufficient contrast. Such target projection imagery acquired at sufficiently high dose, may also be referred to herein as the diagnostic projection imagery λ in order to follow established terminology. However, this naming convention is not to preclude that this projection data λ and its reconstruction V is to be used for tasks other than diagnostics, such as for therapy (in Cath Labs, etc), planning or any other task.

The target anatomical feature TAF relates to the medical purpose of the imaging. Thus, if patient's liver need examining, the target anatomical feature TAF is the liver: it is the liver that is the purpose and object of an abdominal scan to be done.

Each target anatomical feature TAF is in generally associated with an imaging protocol, a set of specifications, that stipulate, preferably as a function of patient bio-characteristics (age, weight, sex, height, BMI, medical record, etc), certain preferred imaging settings, the required image contrast to be achieved, the radiation dose to use for the target anatomical feature TAF for the given purpose, voltage/amperage settings of source X to use, collimation, etc. In short, imaging protocols encapsulate the medical knowledge for any given imaging task, purpose, target anatomical feature TAF, etc.

Before acquisition of such diagnostic projection imagery λ , and for anatomical feature TAF of such low radio-opacity, a volume of contrast agent CA, such as Iodine or other suitable material, is administered to the patient PAT via an administration device ADA (such as a pump). This volume (sometimes called a "bolus") of contrast agent CA then propagates through the patient with blood flow, and accumulates at the target anatomical feature TAF. Ideally, once sufficient contrast agent CA has so accumulated at the targeted anatomical feature TAF, only then should the diagnostic projection imagery at the diagnostic quality be acquired. Thus, the timing for the acquisition by imager IA of the diagnostic projection imagery λ for the target image V is an important consideration as it must be ensured that the concentration of the contrast agent at the target anatomical feature of interest is sufficient. Only then can the reconstructable image domain target volume V be expected to have the required IQ (image quality) as per the protocol or as otherwise prescribed image contrast. If this is not the case, retakes may be required which is to be avoided due to cost, time efforts, increased dose exposure, machine wear (in particular of the anode disk of tube XS), etc. Hence it is a consideration herein to "get" the diagnostic acquisition timing "right first time".

The facilitator system FS envisaged herein facilitates such acquisition at the right timing in contrast assisted imaging protocols. The facilitator FS ensures that, reproducibly and reliably, the diagnostic projection image acquisition is triggered at the right moment when sufficient contrast agent has in fact accumulated at the target anatomical feature TAF.

The facilitator system operates across two phases, an exploratory or preparatory phase PP, and a monitoring phase, which both preceding the target phase at which the target volume V is obtained. All such phases are preceded by an initial phase in which an initial surview image V0 is obtained. This initial image V0 is preferably a 3D (image domain) surview volume, itself reconstructed from a first set of projection imagery. But this first /initial set of projection imagery λ0 is acquired at a lower image quality than the image quality of the projection imagery λ to be acquired later in the target phase and to be used for reconstruction of target image V. In particular, when acquiring λ0, lower radiation dose is incurred as compared to the later dose that will fall due for the diagnostic scan to acquire the diagnostic projection imagery λ. This is to save dose on the patient PAT, and also because the 3D surview volume V0 serves an entirely different purpose than does the target volume V: the purpose of the surview volume/image V0 is essentially one of navigation, as will be expanded upon herein: the surview image V0 is used to find a suitable location to monitor for bolus arrival to ensure imaging for the target anatomical feature TAF commences at the right moment in time, so that target volume V has the expected diagnostic-grade contrast.

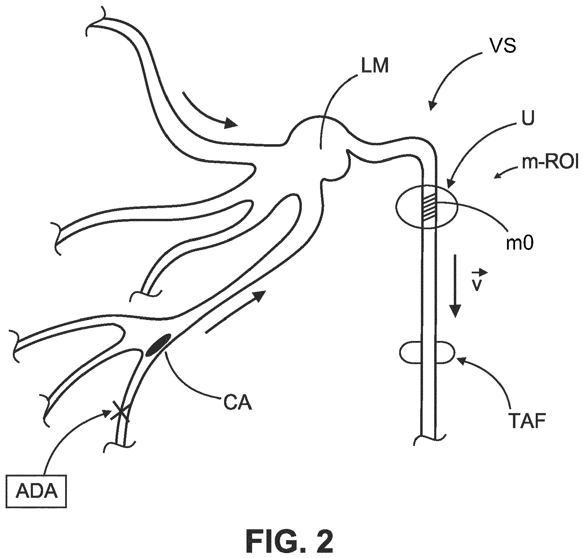

The contrast agent assisted imaging protocol is illustrated schematically in Fig. 2, to which reference is now made, before explaining operation the facilitator system FS in more detail.

As observed earlier, contrast agent CA is to boost image contrast for target structure TAF with poor native radio-opacity. In this connection, and referring now more specifically to Fig. 2, this is a schematic illustration of a part of a blood vessel into which, at an access point (shown at "*X*"), bolus CA is administered by the said contrast agent administration apparatus ADA or other.

A blood vessel, such as a cardiac artery or vein of interest in cardiac imaging, is soft tissue and hence has, as such, poor radio-opacity. It is thus represented at low contrast if one were to use non-contrasted scans. The volume of contrast agent CA travels with the blood flow and propagates therethrough until a concentration of contrast agent accumulates at the target feature TAF at which point the target phase can commence, where the projection imagery for the target volume V at a higher image quality is acquired.

Preferably upstream of the target anatomical feature TAF (blood flow direction is indicated in Fig. 2 by vector *̅v̅*̅), a monitoring region m-ROI is defined by an in-image neighborhood *U* as will be explained in more detail later on. This neighborhood *U* is based on a 3D segmentation *m0* as found in the initial surview image V0. The neighborhood U and the segmentation *m0* may relate to an anatomical landmark (such as a section of the aorta, such as aorta arch, descending part of aorta, etc) at which the monitoring of the contrast agent concentration is to take place. That is, the monitoring region m-ROI may be defined in terms of an anatomic landmark as per medical knowledge initially, although the exact, specific location in the given volume is provided by the facilitator FS.

Thus, the concentration of contrast agent CA is preferably monitored upstream the actual target anatomical feature TAF in order to account for image acquisition latencies for example. Once the concentration as monitored for at the monitoring region m-ROI has reached a certain minimum concentration, as may be measurable by thresholding in a time (*t*) series of tracker imagery *r(t)* (to be explored more fully below), acquisition of the now diagnostic projection data λ for the target volume V may commence as by that time, thanks to the blood flow, the concentration at target anatomical feature TAF can be expected to have reached an acceptable minimum concentration.

The spatial distance between the monitoring region m-ROI and the actual targeted anatomical feature TAF is mainly based on clinical knowledge and/or is determined by characteristics of the patient, knowledge of blood flow velocity, etc. Such medical context information including the m-ROI-TAF distance, which landmarks to use for monitoring region m-ROI, etc, may all be encoded in the mentioned imaging protocol/specification for the imaging task/purpose at hand.

Broadly, facilitator system FS may include a monitoring region finder MRF, configured to quickly, and as said reliably and reproducibly, but also accurately, find the in the surview volume V0 a correct monitoring region m0=m-ROI for a given patient and target anatomical feature TAF. Preferably, operation of the facilitator system FS is such that it integrates well into existing CT workflows.

The monitoring region finder MRF may be implemented as a segmentor SEG, for example based on a machine learning ("ML") model M. Examples for such model M may include artificial neural networks, in particular of the convolutional type. CNNs capable of multi-scale processing such as of U-net architecture may be used. Any other machine learning model may be used. Instead of ML, more classical segmentation techniques may be used such as region growing, shape model-based segmentation, etc.

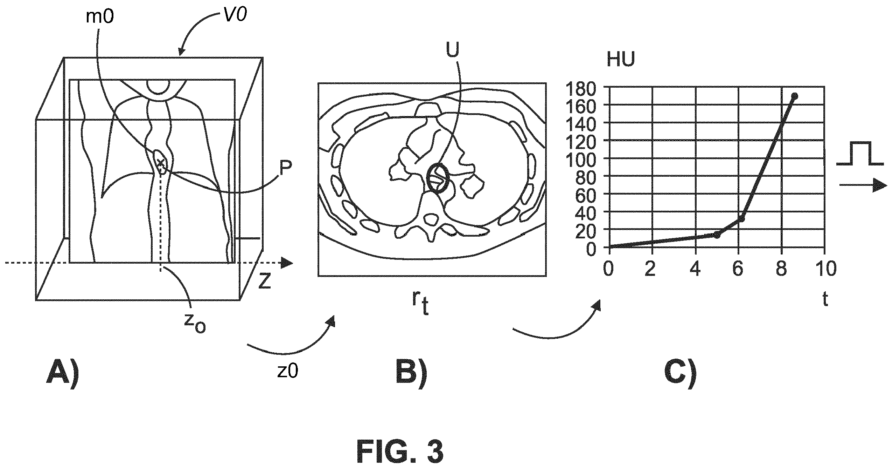

Whilst The monitoring region finder MRF of the facilitator system FS as such may be able to operate fully automatically and provide the monitoring region m-ROI without further/any user input (other than specifying the imaging protocol or target anatomical feature TAF), it is specifically envisaged herein that that the facilitator FS includes a user interface functionality UI that allows user interaction with the estimated m-ROI location m0. Specifically, the facilitator FS invites user-interaction. It is specifically provisioned for herein that certain components of the monitoring region finding procedure can specifically be adjusted by the clinical user at their pleasure, through the user interface UI, such as a graphical user interface (GUI), specifically configured for such user interaction. User request for such adjustment of m0 may be passed on to a bolus monitoring system MS (see below at Fig. 3) via touch screen TS, pointer tool (computer mouse CM, stylus STY), etc. Such user-interaction is preferably envisaged herein as a dynamic real-time experience: upon such user-called-for adjustments in relation to the found monitoring region m-ROI, a re-computation of related components is triggered, and their display may be updated, as many times as the user calls for such changes or adjustments.

Thus, the facilitator system FS, in particular thanks to its user interface functionality UI, allows finding the right monitoring region which in turns facilitates finding the right moment *t=t₀* for the target phase to commence and to control the imaging apparatus IA, to so reliably acquire the higher quality projection imagery λ from which the target volume V can be reconstructed.

Before turning to operation of the user functionality UI in more detail, reference is made first to schematic Fig. 3 which illustrates some basic operational aspects of the proposed facilitator system FS in connection with the (optional) monitoring region finder MRF in cooperation with the bolus monitoring system MS as part of the computing system CS. Specifically, and as illustrated at A), the initial 3D surview volume *V*₀ is segmented for suitable one or more anatomical landmarks that is to serve as the bolus monitoring region m-ROI.

Based on the estimated m-ROI segmentation *m0,* a reference location z0 in the volume is identified which may, for example, be a set location along the rotation/imaging axis Z of the imager IA. Preferably, a reference point P is defined in the 3D segmentation sub-volume *m0* such as a centroid or other. The point P may be projected onto the Z-axis to define reference location *z*0.

A time series of tracker imagery r*ₜ* is then reconstructed from respective sets of low dose projection imagery acquired at suitable sampling interval around reference location *z*0.

In some or each such tracker images r(t), a monitoring neighborhood *U* (shown as a small circle) at B) is defined automatically, based on the reference point P . The neighborhood U around P may be an ellipsoid, sphere, etc (or in 2D view, an ellipse circle, etc), Thanks to the proposed user-interactive feature , an event handler EH may be used to allow user to change any one or more of P, *z*0 or *U,* as required. Thus, it will be understood that the monitoring region m-ROI may be defined by two elements: its location in 3D as given by the reference location *z0*, and the spatial extent for monitoring purposes, as it is represented in the tracker imagery *rt,* which representation is defined by the neighborhood *U.* A change in one at least one of the elements P, z0 or U, will cause an automatic and consent change of the other one or more elements.

In the series of tracker imagery *r(t),* the concentration of the contrast agent arrival is monitored after administration of bolus at the neighborhood, which defines the set monitoring location m-ROI. An example is shown as a contrast curve *c^{U}*(*t*) at C) for a given tracker image and image neighborhood *U.* In such curve, contrast value *HU* (in Hounsfield units) in neighborhood *U* is recorded over time *t.* CA concentration, and hence contrast, is expected to increase over time in a ramp-up phase, to then plateau and reach saturation in plateau phase, and to then drop off in drop-op phase, as the contrast agent washes out. Only the ramp-up phase is shown in Fig. 3.

An image value-based thresholding policy may be used in the monitoring at neighborhood U to trigger acquisition signal for acquisition of the diagnostic projection imagery from which the target volume V is reconstructable. This acquisition of λ should be triggered after the contrast agent concentration (or HU value in the contrast curve *c*) has reached a certain minimum value which may be lower than the expected maximum value at the target anatomical feature TFA, as this is expected to be located slightly downstream of the monitoring region m-ROI/U. Instead of basing the above-described thresholding on monitoring absolute HU values, gradients of HU values may be monitored for instead, or in addition, as required.

However, in some embodiments where latency is not an issue it may be that the monitoring region and the targeted anatomical feature are coinciding in which case the thresholding can be done so that the diagnostic projection image acquisition is triggered at the maximum of concentration values in plateau phase. The maximum may be found by using gradient based methods for example.

The sets of projection imagery acquired at the set sample rate from which the tracker imagery is reconstructed from, is equally of lower quality (lower dose) than the projection imagery λ for the later diagnostic acquisition phase. For example, the image quality may be similar to that of the projection imagery acquired from which the surview image V0 was reconstructed.

The size (field-of-view ["FOV"]) of the surview (also called "scout") image volume V0 is preferably so chosen that it includes not only the target feature TAF, but also at least one of the anatomical landmarks at which the bolus monitoring is supposed to take place. If in doubt, the surview scan may be a whole-body scan, although this will not always be necessary as in some cases a scan over an applicable body region, such as an abdominal scan, head scan, thorax scan, leg scan, etc, may be sufficient.

Whilst contrast is usually very poor in such surview image V0 on account of the low dose, it may nevertheless be sufficient to locate broadly the said landmarks for m-ROI and, optionally, the targeted anatomical feature TAF. The later may also be marked up manually by user (see below).

Once the reference location z0 is found by system FS based on the segmentation *m0,* the imaging geometry may need to be adjusted for example by moving the patient table relative to the gantry so that the tracker imagery around the reference location can be obtained by reconstruction from the series of projection imagery acquired at the mentioned sampling intervals. However, in preferred embodiments, such a repositioning of table or gantry is not necessary, as the distances between target anatomical feature and monitoring location is roughly known, and the surview volume V0 is preferably obtained from the outset to cover a sufficiently large volume. For example, the FOV of the surview scout volume V0 may be linked with an auto-plan, so in general there is no need of a very large FOV surview to collect the anatomical context information, for example for the detection of landmarks, etc. Such auto-plan is a facility that allows detection of the target anatomical feature TAF in the surview image V0, or allows defining the FOV to be used. The auto-plan facility may be implemented by suitable image processing, for example again by segmentation, if required. However, detection and knowledge of location of the TAF may also be provided manually by user, or by whichever means, machine learning based or not. In general, it is assumed herein that the location of the target anatomical feature TAF is known, the main focus in this disclosure being the reliably locating of the monitoring region m-ROI.

The m-ROI segmentation *m0* is a 3D segmentation, that is, it is defined by a 3D sub-volume in the surview image, *m0* ⊂*V₀.* This sub-volume having spatial extensions in all three spatial directions (X,Y,Z) is preferably anatomy aware in that it corresponds, in particular conforms, in shape, size and orientation to the spatial structure of the anatomy at that location. For example, the segmentation *m0* may conform to the spatial structure of the anatomic landmark that is associated with the target anatomy. The segmentation may thus follow at least in part the anatomical/tissue boundary of the landmark. This allows user to quickly verify visually at a glance whether the monitoring region finder MRF-proposed segmentation *m0* makes medical sense.

The, in general, relatively small, segmented anatomy *m0* may be used for the definition of the reference location z0, and, separate therefrom, the definition of the neighborhood U in the tracker slice imagery that pass through the said reference location z0. As mentioned, the reference location z0 may be situated on the imaging axis Z. However, this is not a necessity herein as the initial surview volume V0 may also be provided as a reformat, and the reference location z0 may be a point on any geometrical line for the said reformat, and hence different from the rotational/imaging axis *Z.*

It is of note that dose can be saved, as no separate projection data acquisition is required for the initial locator tracker image r0 as this can be synthesized from the surview volume V0 purely computationally. Specifically, the neighborhood U for use in the "live" tracker imagery as for live monitoring of bolus arrival can already be defined on this proto-tracker image synthesized off the already available surview 3D image V0. No dose needs to be incurred for determining the neighborhood U which represents the spatial extension of the monitoring region of interest m-ROI as representable in the tracker imagery. What is more, both the reference location and the neighborhood U determination can be done in a single step solely based on the surview image V0 and the so synthesized locator tracker image r0. This is more convenient and quicker for the clinical user, and this 1-step operation can still be easily integrated into existing CT workflows as practiced in medical facilities worldwide. It will be understood that use of the locator tracker image r0 is not dependent on its displaying and such displaying is indeed not required herein in all embodiments for the purpose of neighborhood U definition as described in the single step setup.

It will be understood that the above-described monitoring region finder MRF is merely an optional component of the facilitator system FS as is envisaged herein. Indeed, an initial indication of the monitoring region m0 can also be provided purely manually as a segmentation of the surview volume V0. It needn't come necessarily from the monitoring region finder MRF or any other such automated computer system. Indeed, it is the user who may provide it manually by annotation, and may later change their mind, or another clinical user has provided an indication of the location of the monitoring region, etc.

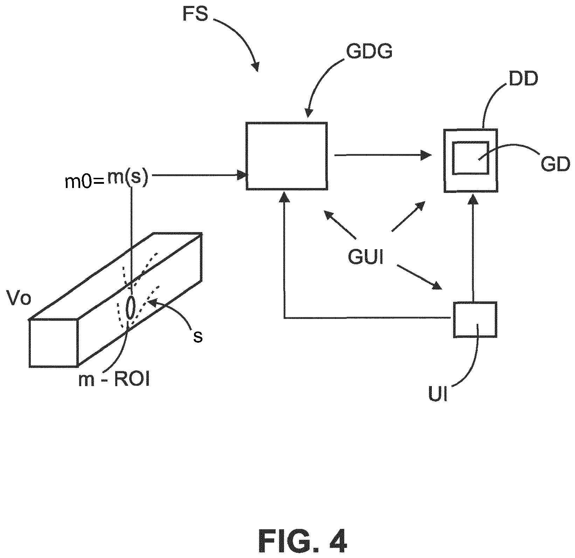

However, what is envisaged herein for the facilitator system FS to include is a graphics display generator GDG as schematically shown in the block diagram of Fig. 4. Graphics display generator GDG is operable to generate, on display device DD, a graphics display GD. The graphics display GD includes a visualization of at least parts of the segmentations of the vessel in question, such as of the aorta, and a graphical indication SL of the current location of the monitoring region, whichever way it may have been supplied either through any user, or by a computerized automated system, such as the monitoring region finder MRF.

The graphics display generator GDG assists (clinical) user in finding interactively the correct monitoring region by repositioning the graphical indication SL. Graphics display generator GDG allows user varying, through a supported user interface UI, the current location of the graphical indicator SL for monitoring region. The graphical indication SL is "anatomy aware" and is dynamically adapted to fit the current anatomical environment as per the segmented vessels. The Optionally, additional useful contextual information (such as landmarks, related distance, expected bolus arrival time, etc) is displayed to correspond to current position of graphical indication SL as will be described herein in more detail below.

The graphics display generator, the display device DD including visualization of the graphics display GD thereon, and the user input device UI interact to form an interactive graphical user interface GUI as is preferred herein. As shown in Fig. 4, graphics display generator GDG receives as input a 3D surview volume with the segmentation s(VS) of a vessel of interest VS, and a definition D of the currently marked up location for monitoring region. This initial definition D *=m0,* whichever way obtained, may include a single 3D co-ordinate or a set of such co-ordinates in the 3D segmented surview volume V0, such as any one or more of reference point P, the neighborhood U around P, and the related z0 position, as illustrated in Fig. 3. A view of volume V0 and at least a part of the vessel segmentation s=s(VS) is rendered for display, including the graphical indicator SL, displayed to mark up the current location of monitoring region m-ROI based on received definition *m0*=D*.* The graphical indicator SL may be rendered as circle, ellipse, or other mark-up symbol capable or area delimitation. Preferably the shape and orientation are such that graphical indicator SL is withing the area confines of the segmentation.

With further, and in more depth reference to vessel segmentation, in some embodiments of the proposed approach, segmentation of vessel(s) is done depending on the anatomy to be scanned (aorta for heart, pulmonary artery for liver or abdominal exams etc.). This may be prescribed in the imaging protocol as mentioned earlier. The segmentation s is based on the low dose surview image(s), as opposed to high(er)-dose images to be used later in the diagnostic scan. Displaying vessel segmentation in addition to the bolus ROI (such as represented by a circle of other geometric Fig) may help, in particular guide, user in placing m-ROIs with greater confidence in a clinically appropriate manner. For example, and in some embodiments, this guiding function is facilitated by the displayed vessel segmentation further including a segmentation(s) of calcification(s) in the vessel, which are typically the regions one may want to avoid when placing bolus m-ROIs. The surview image may be thus recorded with an imager configured for spectral imaging (such as dual energy or any other) and the imagery is spectrally processed by a material decomposition algorithm for better definition of the calcified wall portions. Calcified wall portions w of the vessel may be rendered graphically different from wall portions without calcifications.

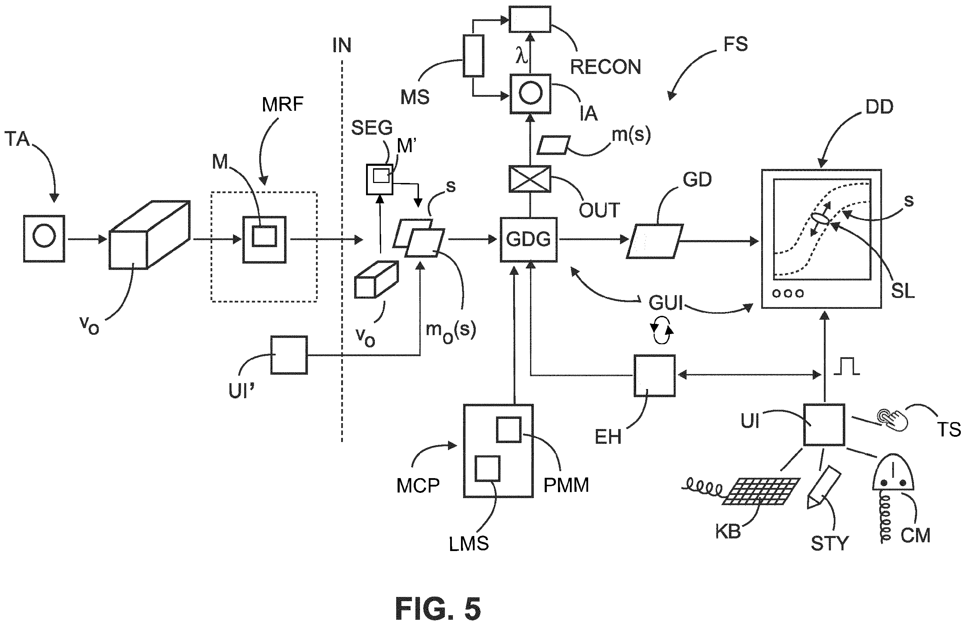

Turning now to the block diagram of Fig. 5, this shows the operation of the graphics display generator as part of the facilitator FS in more detail. Proceeding from left to right, the imaging apparatus is operable to acquire as described the low dose surview 3D volume V0. In some less preferred embodiments, 2D surview may also be contemplated, however the technical-clinical benefits of the proposed facilitator FS are best brought to the fore in the context of 3D surview imagery.

The surview 3D volume V0 may be supplied by the imager IA, preferably in an onlinesetting, although later retrieval from an image database is not excluded herein.

The monitoring region finder MRF may provide the initial segmentation *m0* for the m-ROI. The monitoring region finder MRF may or may not be integrated into the facilitator FS, but, in either case, is optional. One possible detection/output from the monitoring region finder MRF could also be one or more landmarks LM, around which the m-ROI circle may be placed

Indeed, in some cases the monitoring region finder MRF is an outside system, with no functional connection as such to the facilitator FS. But of course, facilitator FS may well be interfaced via wireless or wired network connection to the monitoring region finder MRF, such as in a hospitalinformation system, to form a broader imaging-support arrangement. The monitoring region finder MRF may be implemented for example in a Cloud system, to process imagery produced by imager(s) IA, and these are then segmented on demand or automatically. The segmented volumes V0 may then be stored, and may be loaded by facilitator system FS if and when needed for viewing and monitoring region adjustment. In whichever embodiment, the monitoring region finder MRF may be a machine learning model M powered segmentor component to segment in this low quality (high noise) 3D volume for the initial spatial definition D of the monitoring region therein.

Optionally, it may be that the initial definition D=m0(s)=m0 of location of the monitoring region in the segmentation s is provided manually by a user, by whichever means, such as by leaving an annotation as applied through a suitable user interface UI'. Of course, the monitoring region finder MRF may be entirely dispensed with.

The segmentation of the vessel VS of interest's structure in surview volume **VO** may be provided by a standalone (vessel) segmentor SEG, or the segmentor SEG may be part of the monitoring region finder MR. Even if monitoring region finder MRF is used, may vessel segmentor SEG may still be a standalone component and need not be associated with optional monitoring region finder MRF. In some embodiments, the vessel segmentor SEG is part of the facilitator system.

The segmentor SEG that is to provide the vessel segmentation s(VS) may be ML powered (for, example deep learning), or shape model based (MBS), or based on any other segmentation algorithm, such as region growing, etc. In either case, it is configured to cope with the low SNR in the low dose surview image to achieve good segmentation. As is the case for the initial segmentation m0 of the initial m-ROI, the vessel segmentation s= s(VS) is a 3D segmentation, so forms a sub-volume in surview volume V0. In embodiments, vessel segmentation SEG may be configured to transfer contrast segmentations from spectral CT images to virtual non-contrast images. ML based methods can be used. Segmentor SEG may use an ML model M', such as an artificial neural network (NN), in particular of the convolution type (CNN). NNs or other models configured for multiscale processing may be used, such as NN models of U-net architecture, or other bottleneck type models that use convolutional operators followed by deconvolutional operators. Such models include, for example, the said U-net architecture or its cognates, as described by O. Ronneberger et al in "U-Net: Convolutional Networks for Biomedical Image Segmentation", available online on arXiv repository under citation code arXiv: 1505.04597 (2015). The segmentor SEG may also be conjured to extract a centerline CL and/ radius of the vessel (such as aorta) segmentation s. This can be done using existing skeletonization or distance transform methods. The centerline CL can be visualized, as will be explained in more detail below.

Alternatively, no such segmentor SEG is used, but the vessel segmentation is provided instead by user, based on 3D annotation, although this may be cumbersome and time consuming.

The blood vessel VS, whichever way segmented, may represent all or a part of the aorta for example, or it may be part or whole of any other vessel that is of interest for the medical task at hand, and through which the bolus is expected to travel.

The vessel segmentation s(VS) in the surview volume V0, and the initial definition m0 of the monitoring region location therein, are then processed by graphics display generator GDG to generate the graphics display GD for view on the display device DD. The graphics display generator GDG controls, through a suitable video circuitry interface, display device DD to effect displaying of the graphics display GD on the display device DD. The graphics display GD is schematically represented to the right of Fig. 5. The graphics display GD includes visualization of the segmentations s=s(VS), or parts thereof, and a visualization, within the area of the segmentation s, of the monitoring region indicator SL based on the initial location definition m0.

Upon displaying on the display device DD of the said graphics display GD, user may use a user input device UI, such as a keyboard KB, a stylus STY or a computer mouse CM, or any other pointer tool to request modification the currently indicated monitoring region as per the graphical indicator widget SL. Alternatively, the graphics display generator GDG may interact with the display device DD to support a touch screen TS facility as another embodiments of user interface UI facility through which user can input the modification request for the currently indicated monitoring region. In some of the mentioned UI embodiments such as pointer tool STY, CM or the said touchscreen TS functionality, user may interact with indicator widget SL to request repositioning and/or resizing the currently indicted monitoring region. To this end, and preferred herein, indicator is configured herein as a sliding element SL that can be slid along the segmentation s so as to allow the user to re-position, based on their medical knowledge, the currently proposed location of the monitoring region to more appropriate location. It has been found, that this type of sliding element provides immediate visual feedback and can support user even in time critical and stressful situation (such as in a trauma room or busy clinical settings with overwhelming workloads, staff shortages, etc) to quickly achieve satisfactory monitoring location definition. In addition, the sliding element allows for a quick and intuitive input of such re-positioning request. Thus, throughout most of the present disclosure, indicator SL will be referred to herein as slider element or simply "the slider" SL. The slider SL may also support request for re-orientation and/ or resizing by click-and-drag actions for example if required. Preferably however, slider SL is resized/reoriented automatically and dynamically by graphics display generator GDG, for example, as slider SL is slid along segmentation s (such as along centerline of the segmentation s), or when user requests a change of view rendering/reformatting of volume, etc, as will be described in more detail below.

Once such a user request for position adjustment through the user input UI is received by graphical display generator, event handler EH is operative to capture this request-for- adjustment event, and passes this on to the graphics display generator GDG. The event may include a specification of the nature of the request, for example the next intended position of slider SL, its size, and so forth. Alternatively, the event may be mapped to such as specification. Graphics display generator GDG processes the event specification to update the graphics display accordingly. The updated graphics display GDG now indicates the new position of the monitoring region by modification of the SL elements position along the segmentation. A geometry of slider element SL may be modified accordingly when rendered for display at the new position, as will be described below in more detail.

The user requested adjustments of slider SL's position can be done many times over. Once the user is convinced that a suitable position has been found, the so indicated position of m-ROI by slider SL can be dispatched via an output interface OUT as the final monitoring position m(s). This final position m(s) may then be passed on through a suitable control interface to the imaging apparatus, such as to its operator console OC to initiate the monitoring phase which may be coordinated by a (bolus) monitoring system MS. In this monitoring phase, based on the now indicated position (and extent) of the monitoring region m-ROI, the imaging apparatus IA is instructed by monitoring system MS to acquire a stream of tracker projection data around this region m-ROI. As coordinated by monitoring system MS , tomographic reconstructor RECON reconstructs therefrom a stream of tracker imagery which is monitored by an in-image monitoring unit of monitoring system MS for voxel value changes at the monitoring region. For example, image values such as HU values as per the stream of tracker imagery are thresholded. Any other monitoring policy may be used instead or in combination with thresholding. Image value changes are a telltale for the arrival of the bolus. Once the monitoring unit of monitoring system MS concludes, based on the thresholding or on any other applicable such monitoring policy, that bolus has indeed arrived at the indicated location m(s), a new signal is issued by monitoring system MS for the imager IA to acquire a new set of projection data, but this time at diagnostic dose - higher than the dose that was used for the surview scan V0 or the tracker imagery r(t). From the diagnostic projection data a fully contrasted target volume V may then be reconstructed by reconstructor RECON. The target volume V may then be displayed on the display device DD or on any other display device, in order to support user in the clinical objective/purpose of the imaging. The purpose may be any one or more of therapeutic, diagnostic, for planning, medical analysis, etc or any other depending on the clinical context. The target volume V can be processed (eg, analyzed, etc,), stored, dispatched as required.

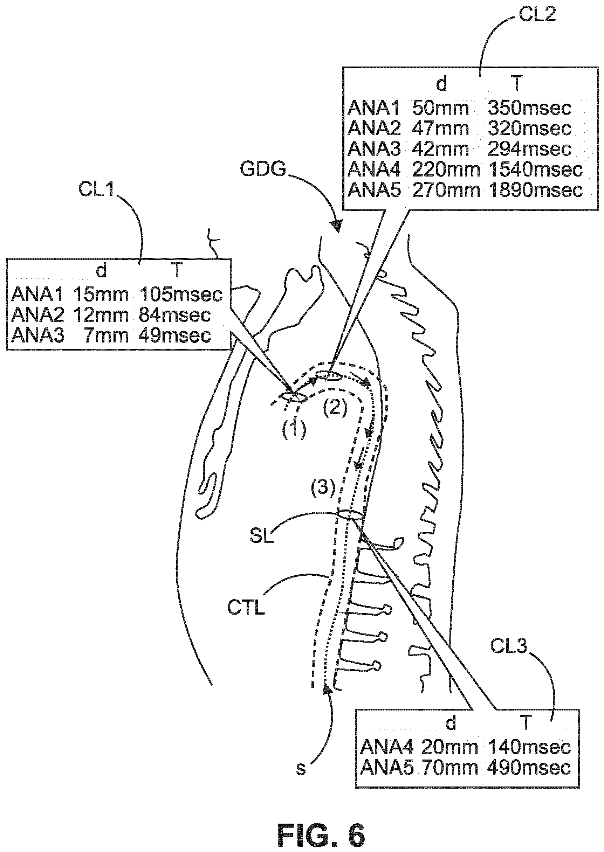

Fig. 6 is an illustration of a graphics display GD generatable by the graphics display generator GDG according to one example embodiment.

The graphics display GD includes a view of volume V, either in a 3D rendering (eg, isosurfaces, etc), or as a 2D sectional view in a user selectable section plane as shown in Fig. 6, along with a visualization of segmentation s=s(SV) of the vessel VS of interest S. In the example as shown in Fig. 6, the vessel of interest is a part of the aorta shown in sagittal view. As a third visualized component, there is a graphical widget of the slider SL, rendered as an elipse, but it can be of any shape or size, so long as it remains inside the arealvolume marked by segmentation s of the vessel of interest VS.

The view can be in any plane, and is not necessary sagittal such illustrated in the Fig. 6. Views on frontal plane, lateral plane, are also envisaged herein as standard views, and so are non-standard re-formats in planes different from the standard planes as defined in image domain. One such a change if view, be it a re-format or standard, is requested, visual elements of the graphics display are adapted accordingly. For example, the segmented aorta and the slider symbol SL are re-rendered in correct and consistent geometrical perspective. The user can request a change of view at any time at his or her leisure by issuing a request event through UI. Event handler EH intercepts this and mediates the view change. To this end, graphics display generator may include a geometrical graphical view generator that is aware of the geometry of the imaging domain and is configured to apply geometrical operations of projective geometry to effect the re-rendering of graphics display into any view as desired, with the said re-rendering of visual components including segmentation s and slider SL as mentioned earlier.

Segmentation borders of the aorta VS are indicated dashed lines, and such rendering is indeed envisaged. Thus, the segmentation preferably includes a distinct visual modulation to represent wall W of the vessel VS, such as of aorta. Such a distinct visual rendering of vessel's wall portion W has been found to better assist user in placement/positing of the slider element SL for the defined monitoring region. Realistic and clinically meaningful spatial specifications of monitoring region m-ROI can thus be quickly achieved even for novice users, or those in stress situations. The visual modulation of wall portion may differentiate between calcified and non-calcified vessel wall portions, as mentioned above.

The graphics display generator GDG may further include a failsafe measure that assists the user in yet better positioning the slider FL for m-ROI specification. This may be achieved by the graphics display generator GDG constricting the apparent motion of the slider SL during the adjustments into one single dimension, mainly to slide along the direction of the vessel, which may also include curved portions.

More specifically, and in embodiments, the slider locked into course of the vessel as may be defined by tangents to its center line/curve. Thus, the slider SL may only be slidable/ moveable in a motion along tangents of the center line CL of the vessel segmentation s. It is the center line CL that may also be visualized in the graphics display GD, for example as indicated by a dotted line in Fig. 6. Any line styles can be used herein for the center line, and for the wall W indication of the vessel segmentation s, the chosen dashed and dotted modulations merely being illustrative embodiments herein. Separate and distinct visual modulation by color- hue- or grey value coding, to set wall W and/or center line apart from the remaining body of the vessel segmentation may be used in addition or instead to line style modulation.

Adjustment requests may lead to the slide element SL being dragged from one position, indicated as (1), to a next position (2) and then (3), as illustrated by bracketed numbering and related arrows in Fig. 6.

The slider element SL itself may have any suitable form or shape. Preferably the shape is dependent with on the rendered view and remains consistent with view changes. For example, slider may be rendered as an ellipse, circle or may have any other shape. The shape may change as user requests new view, such as from sagittal to transverse, etc. For example, the shape may be an ellipse in sagittal plane view, but may change over into a circle when user request view in transverse plane instead. View changes may also be requested by user interface UI. For example, a pop-up drop-down menu widget may appear when operating the pointer tool in a pre-defined manner (such as mouse right click, or other) and user may then select the new view, which is then rendered by graphics display generator GDG on display device DD.

User can also request a resize adjustment or change in orientation of slider SL using user interface UI. Size adjustment may be restricted by the area *d* marked out by the vessel segmentation of the vessel. In a preferred embodiment, as the user requests sliding of the slider element along the center line SL and within the segmented area, size of the slider SL is automatically adjusted to conform with the diameter or width of the segmentation s. In other words, the slider element SL in a given geometrical shape (such as an ellipse) has its size adapted to a maximum size, but one that still remains within the segmented wall W. For example, a circle is inscribed with a specific diameter around the segmented centerline and which circle is, preferably completely, inside the segmented vessel area s. Thus, the slider SL appears to contact at all times the wall portion W of the segmentation s as it is slid along the center line SL, the sliding caused by the user issuing through user input interface UI corresponding position adjustment requests. To the user, this auto-conformation functionality of slider SL with the geometry of the vessel segmentation may appear as a dynamical inflation or deflation of the slider SL as this dragged through the vessel segmentations with it varying width (cross section). The auto-conformation of slider with vessel segmentation geometry is also provided irrespective of the slider SL being slid along a curved or straight section of the segmentation s(VS),

The sliding of the sliding element along the tangent of the center line SL may be effected simply by a keyboard strokes as one possible embodiment of user interface UI envisaged herein. Thus, in such or similar embodiments, user is invited to press either one of two designated keyboard keys for issuing a slider SL reposition event. For example, each of the keys may indicate one of two directions of movement for slider SL, up or down in the view as per Fig. 6, which may correspond to left or right in other views, etc. In addition or instead, the user interface may allow user to increase/decrease diameter of the bolus ROI circle. For example, this may be achieved in a touch-screen-embodiment by user performing certain pre-defined gestures. Alternatively, in a keyboard-based embodiment, user may be hitting a set of pre-defined keys to request such change in the displayed m-ROI size. For example, the set of "plus/minus" keys may aptly assigned to allow user requesting such as change in m-ROI size.

In some embodiments, the adjustment of the position of slider SL is restricted to occur only tangentially to the center CL of the segmentation. The motion of the slider is thus locked onto the course of the segmentation. Thus, graphics display generator GDG is configured in preferred embodiment to support repositing of the monitoring region m-ROI and hence of the slider SL only along the segmentation. Other repositioning request may be ignored or may be converted to as to maintain the slider SL withing the confines of the segmentation. If the initial definition m0 of then current monitoring region m-ROI violates this policy, the initial definition may be auto-corrected so that the slider SL for this initial position is within the segmentation. The user may be notified of this auto-correction.

Some manners of user interaction as supported by the graphics display generator GDG may include an indication by mechanical input. For example, in touch screen interaction TS, user places their finger on the current position and performs a drag movement across the screen in order to effect the sliding of the sliding element. Other examples include a computer mouse CM or stylus STY event that may indicate such as drag operation. In such mechanical user interface arrangements where the user describes a motion such as by touch screen action, stylus or mouse, etc, the registered motion is analyzed by the event handler EH, and is resolved into components including one that is parallel to the current center line CL tangent at current position of slider SL. The event handler of graphics display generator GDG projects this parallel component of the requested motion onto the tangent of the said center line. It then only this projected parallel component that is used to slide slider SL to the next position. Thus, the graphics display generator GDG effects a motion of slider SL along the tangent only in proportion to the so projected motion component. In this way, by projection of motion components onto instantaneous tangents of center line SL, the apparent motion of the slider can be restricted to occur only along the center line, and is thus being locked onto the segmentation.

As will be understood from the above, the graphics display generator GDG supports a number of restrictions such as auto-conformation of slider SL size to conform geometry of its graphical representation with the width of the segmentation, and the locking feature where requested repositing is locked onto the segmentation's course (eg, center line). These restriction functionalities form useful failsafe measures that as shown in the current view is an option that can be disabled if need be. The sizing of slider SL as function of the instantaneous segmentation width and the locking feature to force lineal reposition help even novice, or under-stress, users to quickly find a realistic bolus monitoring location, thus reducing the likelihood for retakes, increase patient throughput, etc.

However, it will be understood that the locking onto of the sliding motion to the tangent of the vessel segmentation may not be necessarily in all embodiments. In addition, the graphics display generator GDG may include a feature can allow user to disable any one or more of the restrictions in order to more freely place the slide element SL, as may be called for in some instances.

As an additional optional feature, the graphics display generator GDG supports displaying of useful medical contextual information, in addition to the displaying of the segmentation s and the slider SL in or around the segmentation s. The medical context information mentioned may be provided by a medical context information provider MCP. The medical context information provider MCP This may be part of facilitator system FS, or facilitator system FS is configured to suitably interface with such medical context information provider MCP. Medical context information provider MCP may include a landmark segmentor LMS module, and or a physiological modelling machine PMM that is configured to provide the distance d and/or transit time information.

Thus, in some embodiments, and as illustrated in Fig. 6, graphics display generator GDG may operate to cause displaying of medical context information, such as transit time *T* of bolus and/or distances d to certain anatomical landmarks LM, ANAj, (j=1-5 as illustrated) as function of the current slider SL position. Such medical context information may be usefully and contextually apt displayed in information-pop-up widgetry CL1-CL3. Such widgetry (call out type pop-ups are illustrated in Fig. 6), may be generated by the graphics display generator GDG in association with the varying positions (1), (2), (3) of the sliding element SL, with information in the pop-ups CL1-CL3 being updated dynamically. The pop-ups CL1, Cl2, CL3 may include information on distance d to various landmarks LM as measured from the respective ( (1), (2) or (3)) current position of the slider SL, and the expected arrival time T of the bolus. In addition or instead to indicating such time T and distance d information to landmarks LM, the time T and/or distance d may refer to the target anatomic feature TAF to be imaged. The pop-up are invoked at user's request or automatically as slider SL is slid along segmentation. Pop-ups Cl1-Cl3 may be persistent, or timed. If persistent, user may force same into retirement upon issuing a close request, by clicking on the pop-up for example, or the like. If displayed persistently, either on user request automatically, the time and/or distance information is updated automatically and dynamically.

Usefully landmarks LM in particular for cardiac applications may include any one or more of the aortic valve, ostium of the coronary arteries, branching points of renal and hepatic arteries, any is such landmarks LM could be included to facilitate better planning. Knowing the 3D locations of such landmarks enables a useful differentiator of 3D surview based planning, namely the ability to incorporate in the planning true 3D distances between bolus tracking ROIs and relevant landmarks.

As to the transit times, optionally, the physiological modelling machine PMM may be configured to implement hemodynamic models to compute transit time T estimations to the distances d from landmarks. The distance can be determined for the surview volume V0 based on landmark segmentations as provided by landmark segmentor LMS module for example. The hemodynamic models may contain textbook knowledge about flow velocities, or patient specific information such as current heart-rate or blood-pressure together with a model of the patient's vasculature derived from the 3D surview. Using transit time, the bolus tracking locations can be standardized with respect to time delays and the information could be displayed to the technologist while modifying the location of the bolus m-ROI. The distance and transit time information my thus be obtained by a medical context information provider, that my implement such hemodynamically modeling, or that may query through suitable interfacing database to locate such distance and transit time model.

In embodiments, the graphics display GD may include a visualization of certain aspects of the said hemodynamic modelling, such as color-coded streamlines, velocity fields or pressure fields, etc.

The pop-ups CL1-3 can be used with particular benefit in combination with the locking features where motion of slider is locked onto the course of centerline CL. Locking the slider SL into the centerline in this manner affords a guiding function that avoids the time-consuming user interaction to freely place the bolus tracking ROI (typically circle) and allows user instead to "slide" the ROI along the centerline. For example, for each or some possible locations, the distances *d* to relevant landmarks ANAj are displayed in the pop-ups CL1-3. This can help in the standardization of bolus tracking, for example to *"always place the ROI 10 cm downstream of the left coronary ostium for a coronary scan".* In addition, the radius of the bolus tracking ROI can be adjusted dynamically based on the local radius of the aorta VS.

In addition to displaying such pop-ups, the location of the relevant landmarks may be indicated by an indicator widget (box, circle, ellipse, etc). The information in the location of the landmarks LM may have been segmented for already in the surview volume v0 as received, or the facilitator system FS may include the said one or more landmark-segmentor modules LMS, be they ML-based, or MBS-based, or based on whatever technology, so long as segmentor module LMS is suitably configured to cope with the expected low signal-to-noise ratio (SNR), on account of the low dose acquisition. Again, as with the segmentation s of vessel, the segmentation of landmarks LM are in 3D, so are 3D subsets of the surview volume V0. Segmentation of such anatomical landmarks LM could be achieved by employing ML based landmark detection techniques, by the segmentation of blobs around the landmarks, or by using more traditional image processing approaches which take into consideration the geometrical and anatomical properties of the landmarks relative to the aorta or other vessel of interest, such as in MBS shape segmentation techniques.

Once a suitable placement as per current position of slider SL has been found, the user can indicate this by issuing a specific event through user interface UI, for example by hitting a specific key, or by performing a specific gesture in the touch screen embodiment, a click event, etc. In either case, the extent and current position of slider SL is deemed indicative the monitoring region m-ROI, and a specification of the said current position/extent (size) is dispatched through output port OUT to the imaging apparatus and monitoring system MS to initiate, as described above, acquisition of the tracker imagery to facilitate obtaining the final, fully contrasted, image V.

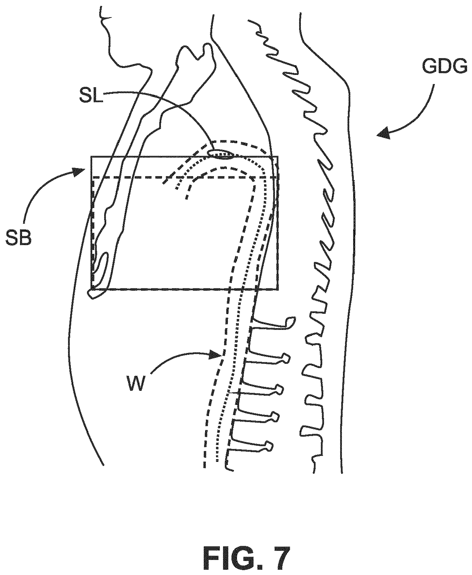

Fig. 7 shows a view of a second embodiment of graphics display GD. In addition to the slider element SL, a scan box SB area is indicated for example by a rectangular area in dashed, dotted, solid, or whichever line style. This scan box visualization SB is co-adjusted with the location of the sliding element as indicated in Fig. 2. In one instant, the scan box, shown in solid lines, is associated with one location of the slider element SL But once slider SL is moved, so is the scan box as shown at the next position in dashed lines.

Preferably, one end of the scan box, for example one of its edges/planes, passes through the currently indicated slider element SL. The end potions of scan box demark the 3D subset in image domain along rotation/imaging axis Z direction for which tracker projection imagery r(t) is to be acquired once a suitable location of the monitoring region (as indicated by slider SL) is found. The monitoring region in included in the scan box, preferably at its edge/end portions. By default, and as per applicable scan protocol, the scan box is chosen in volume so as to include the TAF and, preferably, one or more landmarks ANAj. Graphically indicating the scan box SB in this manner, that is, concurrently with the underlying segmentation and slider SL position allows user to yet better assess or understand the imaging operation that needs to be done.

Thus, what is envisaged herein in embodiments, is that the planning of the bolus tracking m-ROI can be combined with a synchronous planning of the reconstruction box SB. Since it is usually preferred to start the scan at the location of the bolus tracking, in order not having to reposition the table before the scan can start, the manipulation of the ROI location can be coupled with the definition of the superior or inferior reconstruction box limit.

Depending on the selected protocol and the target anatomy, the slider SL for bolus m-ROI may be placed by user using UI around the center-line CL of the aortic walls W at the most superior or inferior end of the plan box SB. Such placement ideally initiates the scan at the respective start/end position of the target organ TAF, and avoids the table movement needed to cover the entire target organ otherwise.

The scan box size, its inferior and superior ends can be estimated by an ML model M as may be used on the monitoring region finder MRF, that only estimates the m-ROI m0, but also the applicable scan box size. Alternatively, the scan box size is specified in the imaging protocols, and retrieved from graphics display generator GDG and used to render the scan box as shown in Fig. 7. Alternatively, still the scan box SB is marked-out graphically manually by user using user interface UI. As a further alternative, the vessel segmentor SEG estimates the scan box, alongside the vessel segmentation s(VS). The imaging protocol information may be used as context information for this estimation.

In some embodiments, the act of placing the slider SL in a final position may automatically initiates (by interaction with monitoring system MS) the scan at the respective start/end position of the target organ as per the scan box SB, and thus avoids the table PS movement that may be needed to cover the entire target organ TAF otherwise.

Thus, in embodiments, reconstruction box SB planning can be coupled to the adjustment of the bolus tracking location *m* as per user interface UI.

It will be appreciated from the disclosure herein that Figs 6,7 are highly schematic and are merely illustrative of embodiments. Thus, the specific configuration and rendering of slider SL, vessel/organ segmentation s, center line CL, wall W, pop-ups CL1-3 etc, are exemplary, and any modifications of any of these elements are also envisaged herein, as long as they support the above described advantages and guiding functions of allowing use to quickly, consistently, reproducibly, and accurately place the monitoring region of interest m-ROI in a (preferably 3D) surview scan.

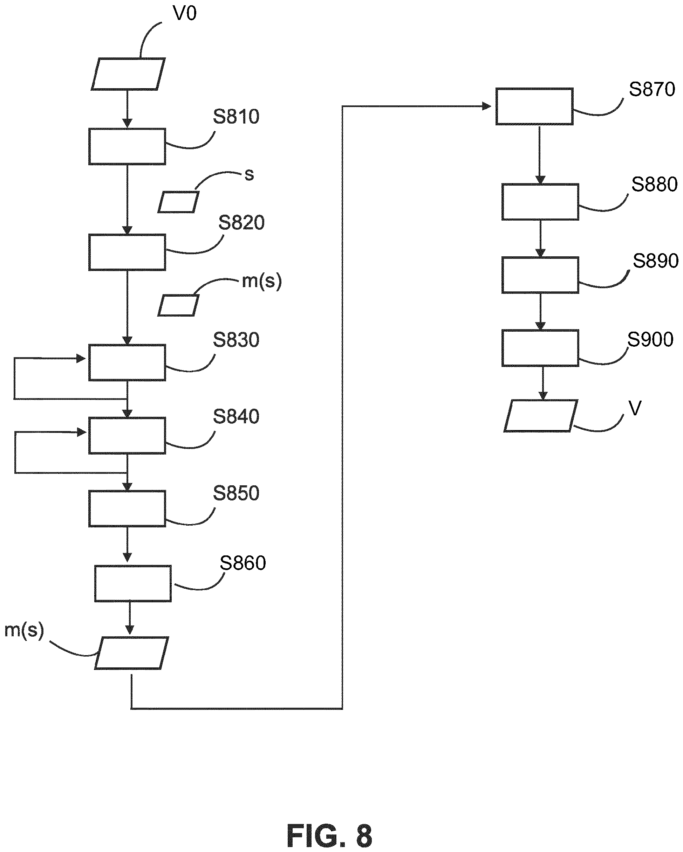

Reference is now made to Fig. 8 which shows a flow chart of a method of facilitating contrast agent assisted image protocols, in particular in finding and adjusting a position and extent of a monitoring region, based on a graphics display generator and its graphics display as explained above. However, it will be understood that the described steps below are not necessarily tied to the system as described above.

At step S810 a segmentation s of a target vessel VS in the surview low dose reconstructed volume V0 is received, along with an initial indication for a monitoring location in respect of this segmentation. The surview low dose reconstructed volume V0 may also be so received.

In step S820 the segmentation with the indication of the current monitoring location is visualized in a graphics display against the backdrop of a view of the surview volume V. This can be done as overlay graphics for example on the surview image. The current position for the bolus monitoring region may be indicated by slider graphic element LS, having a shape (such as an ellipse, circle, etc) that conforms in size with width of the segmentation at the current position. The slider S GUI widget is preferably inside the segmentation.

At step S830 an event handler listens to a user issued request for changing the position of the current monitoring region.

If such a request is received, the monitoring location is changed accordingly in step S840 by causing the slider to be repositioned.

Preferably, the user request for change of slider position as received at S840 is modified so that the apparent motion of the graphical slide element from the current position on segmentation to the newly requested one is constricted to move only along the segmentation. Thus, the reposition motion is locked onto a respective tangential direction of the segmentation center line. Thus, the slider is caused to move on and along the centerline to the new position. Any other directional component in the request, is transformed (eg, by projection) to act only along center line of elongated segmentation arealvolume of the vessel.

Optionally, and in addiction, in step S840 a size (eg, width) of a shape of graphical indicator for the monitoring region is auto-adjusted to follow and vary with the width of the segmentation so that the graphical slider SL remains within the perimeters of the segmentation at all times. Preferably, the size is enlarged or shrunk as a function of possibly varying segmentation width, so that slider SL slides along and touches from within boundary portion of the segmentation as rendered in the visualization.

At step S850 the graphics display is rendered to include additional visualization(s), such as any one or more of: scan box for later tracker imagery/ information on bolus arrival/transit and/or distance to landmarks/ separate graphical indication (outlining, highlighting, etc) of vessel wall, as required.

The landmark distance and transit/arrival time are relative to the current position of monitoring region as per set slider.

Scan box may be indicated as graphical indicator such as a rectangle or square. Scan box marks out the region domain for which projection data is to be acquired for the track imagery and/or the final contrasted image acquisition. Scan box volume include the monitoring region, and the target anatomical feature TAF, and, optionally, one or more landmarks. This scan box visualization is preferably rendered to co-vary with change of position requests for the monitoring region. Preferably one of scan box's ends, for example one of the sides of edges of the rectangle representation, is rendered and positioned so as to pass through the currently indicated position of the slider element.

Step S850 of rendering the graphics display may further include a dedicated visualization (separate from the visualization of the main segmentation body), of the vessel boundary.

After one or more requests for changing the position of the monitoring location, a satisfactory location/size/orientation for monitoring region m-ROI is established, a corresponding approval signal is issued at step S860 and the co-ordinates of the currently indicated position of slider SL are passed on to imaging apparatus to request acquisition of low dose tracker projection imagery and reconstruction thereof at the said position, to produce a series of tracker imagery at step S870 to so allow monitoring for image values changes due to arrival of bolus at the said position.

At step S880, tracker imagery at the indicated location is monitored for changes in image values indicative of imminent bolus arrival.

Once it is concluded at step S870 that sufficient amounts of contrast has accumulated to that at the downstream target anatomical feature TAF contrast agent concentration has entered plateau phase PP, second signal for projection data acquisition is issued at step S890 to acquire fully contrasted diagnostic projection imagery at higher dose from which a contrasted target image volume V can be reconstructed at step S900.

The segmentor SEG that may be used to compute the (vessel VS) segmentation *s* of the surview image V0 may be ML based. In ML approaches, an ML model (such as a convolutional neural network (CNN), or other) is trained based on training data. The training data may include surview input imagery x. The surview input imagery x, as indeed any surview image, has very poor contrast, and it may not be always possible, even for a human expert, to annotate for the ground-truth m-ROIs reliably, or at all. In such situations, a training data generator system may be operable to generate pairs of training data (x,y) based on noise simulation as follows. A set of extant historic 3D diagnostic images (spectral or nonspectral imagery) of the general body part of interest, for which the model is to be trained, is localized in a database query in medical database, such as in a PACS, or other. In such diagnostic images, the annotation can be achieved easily by the clinical expert to define location and extent of the m-ROIs. Then, the high IQ (image quality) of the diagnostic images, that were used for the annotation, is artificially reduced by simulating noise and adding this noise to the diagnostic images, thereby simulating the low dose effect in surview imaging, and thus obtaining artificially generated samples that represent, in good approximation, instances of surview volumes. The noise corrupted 3D image samples thus may serve as the training input x, whilst the annotation on the high-quality imagery serves as the associated ground truth y. Thus, the training data generator system may include a noise simulator and a noise adder to generate as many pairs of training data as needed, along the lines described above. However, as observed above, the facilitator system FS can be used with any segmentor system, be they ML based or not.

The components of the facilitator system FS may be implemented as one or more software modules, run on one or more general-purpose processing units PU such as a workstation associated with the imager IA, or on a server computer associated with a group of imagers.

Alternatively, some or all components of facilitator system FS may be arranged in hardware such as a suitably programmed microcontroller or microprocessor, such an FPGA (fieldprogrammable-gate-array) or as a hardwired IC chip, an application specific integrated circuitry (ASIC), integrated into the imaging system IA. In a further embodiment still facilitator system FS may be implemented in both, partly in software and partly in hardware.

The different components of facilitator system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solidstate medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. System (FS) for facilitating contrast-agent based tomographic imaging, the system comprising, when in use:
an input interface (IN) for receiving a 3D surview image volume (Vo) acquired by a tomographic imaging apparatus (IA) of at least a part of a patient (PAT) in a preparatory phase prior to a contrast agent assisted imaging phase, the 3D surview image volume including a segmentation (s) for a vessel (VS) through which contrast agent is to pass in the later contrast agent assisted imaging phase;
a graphics display generator (GDG) configured to generate a graphics display (GD) of a graphical user interface (GUI) for display on a display device (DD), the graphics display (GD) including a visualization of the said segmentation and of a slider element (SL) indicative of a reference location (m-ROI) along the segmentation, the reference location suitable for monitoring in the later contrast agent assisted imaging phase for presence of contrast agent in relation to at least one target anatomical feature (TAF); and
an event handler (EH) configured to instruct the graphics display generator (GDG), upon receiving user input, to update the graphics display (GD), so as to cause the slider element (SL) to slide along the segmentation, the slider thereby indicating different such one or more reference locations.

2. System of claim 1, wherein the graphics display generator (GDG) is operable to adapt a spatial extent of the slider element to correspond to, and vary with, a geometrical aspect of the segmentation at the said different reference locations.

3. System of any one of preceding claims, wherein the graphics display generator (GDG) is operable to lock the slider element onto a direction defined by the segmentation.

4. System of claim 3, wherein the said direction is as per a center line of the segmentation.

5. System of any one of preceding claims, with the visualization of the segmentation configured to distinctly represent a wall portion (W) of the vessel (VS).

6. System of any one of the preceding claims, wherein the graphics display generator (GDG) is operable to cause the graphics display to include information on i) a distance of the at least one target anatomical feature (TAF) to a) the reference location of the vessel that corresponds to the current slider location, and/or b) to one or more anatomical landmarks, and/or ii) estimated contrast agent arrival time at the said reference location.

7. System of any one of the preceding claims, including an output interface (OUT) for passing on a user selected one of the one or more reference locations to the imaging apparatus for demarking one end of a subset in image domain in respect of which projection data is to be collected by the imaging apparatus in the imaging phase.

8. System of any one of the preceding claims, wherein graphics display generator (GFDG) is operable to generate the graphics display (GD) to further include a visualization of the said subset, with the slider element positioned at the said one end of the visualization (SB) of the subset.

9. System of any one of the preceding claims 1-8, including an output interface (OUT) for passing on a user selected one of the one or more reference locations to instruct the imaging apparatus (IA) to acquire a first set of projection data whilst contrast agent propagates in patient, and comprising a reconstructor (RECON) to reconstruct, based on the projection data, and at a first image quality, sectional tracker imagery in a plane passing through the selected reference location.

10. System of any one of the preceding claims 6-8, where the arrival time is based on hemodynamical modelling.

11. System of any one of preceding claims, wherein the said vessel includes at least a part of the aorta.

12. Method for facilitating contrast-agent based tomographic imaging, comprising:
receiving (S810) a 3D surview image volume (Vo) acquired by a tomographic imaging apparatus (IA) of at least a part of a patient (PAT) in a preparatory phase prior to a contrast agent assisted imaging phase, the 3D surview image volume including a segmentation (s) for a vessel (VS) through which contrast agent is to pass in the later contrast agent assisted imaging phase;
generating (S820) a graphics display (GD) of a graphical user interface (GUI) for display on a display device (DD), the graphics display (GD) including a visualization of the said segmentation and of a slider element (SL) indicative of a reference location (m-ROI) along the segmentation, the reference location suitable for monitoring in the later contrast agent assisted imaging phase for presence of contrast agent in relation to at least one target anatomical feature (TAF); and
instructing (S840) the graphics display generator (GDG), upon receiving user input, to update the graphics display (GD), so as to cause the slider element (SL) to slide along the segmentation, the slider thereby indicating different such one or more reference locations.

13. Imaging arrangement (IAR) comprising the system (SYS) of any one of the preceding claims, and one of more of: the imaging apparatus (IA), the display device (DD), a contrast agent administration apparatus (ADA) for administering contrast agent, a segmentor (SEG) configured to provide the segmentation (s), a bolus monitoring system (MS).

14. A computer program element, which, when being executed by at least one processing unit, is adapted to cause the processing unit to perform the method as per of claim 12.

15. At least one computer readable medium having stored thereon the program element of claim 14.
